# EUROPEAN PATENT APPLICATION

(11) **EP 4 278 971 A1**
(43) Date of publication of application: **22.11.2023**
(21) Application number: 22174066.5
(22) Date of filing: 18.05.2022
(51) Int. Cl.: A61B 5/1495, A61B 5/1455

(54) **OPTICAL MEASUREMENT METHOD USING WAVELENGTH SPECIFIC CORRECTION FACTORS AND CORRESPONDING OPTICAL SENSOR**

(71) Applicant: Universität Zürich, 8006 Zürich (CH)
(72) Inventor: KALYANOV, Alexander, 5507 Mellingen (CH); KLEISER, Stefan, 79639 Grenzach-Wyhlen (DE); NÜSSLI, Anais, 9500 Wil SG (CH); OSTOJIC, Daniel, 8046 Zürich (CH); WOLF, Martin, 8038 Zürich (CH)
(74) Representative: Mertzlufft-Paufler, Cornelius

(57) **Abstract**

For improving the accuracy and reliability of an optical measurement performed with an optical sensor (1), which may be designed as an optical NIRS-sensor in particular, it is suggested to determine at least two different wavelength specific correction factors (c₁(λₘ₁), c₂(λₘ₂)). The correction factors are applied individually for correcting measurement results, in particular measured primary intensities, which have been obtained using a set of at least two distinct measurement wavelengths (λₘ₁ ≠ λₘ₂) forming a primary radiation that is propagating along a primary optical path (22). This concept is particularly useful for mitigating the detrimental effects of superficial absorbers on skin, which are illuminated by the primary radiation. The concept may be implemented by using separate auxiliary light sources (4) which emit an auxiliary light comprising at least two auxiliary wavelengths, which must not necessarily match the measurement wavelengths, however; or using at least one auxiliary detector (7) which also allows measurement of a secondary radiation that has propagated along a secondary optical path (23) (Figure 3).

## Description

The present disclosure relates to the field of optical near infrared spectroscopy (NIRS) but can also be employed in other optical measurements. In detail, the disclosure concerns a method for quantitatively determining at least one optical or physiological parameter in a medium such as tissue, using an optical sensor. The method comprises the following steps: irradiating the medium with a primary radiation comprising at least two distinct measurement wavelengths (λₘ₁ ≠ λₘ₂) which are emitted by at least one primary light source (i.e., in particular by at least two primary light sources); measuring primary intensities (Iₘ₁, Iₘ₂) of the primary radiation for each of said at least two measurement wavelengths (λₘ₁, λₘ₂) after said primary radiation has propagated through said medium along a respective primary optical path.

In addition, the disclosure also concerns such an optical sensor, which can be configured as a NIRS-sensor or as an oximeter, for measuring an optical or physiological parameter in a scattering medium such as tissue. This sensor comprises at least one primary light source for emitting a primary radiation comprising at least two distinct measurement wavelengths (λₘ₁, Xₘ₂), and at least one primary detector for detecting primary intensities (Iₘ₁, Iₘ₂) of the primary radiation after said primary radiation has propagated through said medium along a primary optical path; and an electronic unit, which is configured for computing an estimate of the at least one optical or physiological parameter based on said measured primary intensities.

Spectrophotometry allows determination of optical properties such as absorption and scattering coefficients by illuminating a specimen and determining the attenuation in light intensity over distance. Oximeters are apparatuses that make use of spectrophotometry for determining the (arterial, venous, or mixed) oxygenation of blood in tissue.

The method introduced above is widely employed in optical NIRS measurements for measuring the oxygenation level in cerebral tissue. In such a situation, the optical sensor as described above is placed with its lower contact surface onto the skin of the skull. The sensor emits infrared light, comprising the at least two distinct measurement wavelengths, into the skull. The infrared light penetrates the cranial bones and is scattered by the tissue and also partially absorbed by chromophores such as oxy- and deoxyhemoglobin.

The light thus propagates by scattering through the tissue and can also re-exit the skull at the location of the primary detector of the sensor, as the light is not only forward-scattered but also back-scattered. The primary detector can thus measure an intensity that results from all the photons that have been emitted by the primary light source and which have reached the primary detector, after having traveled through the tissue. As these photons take myriads of different optical paths through the tissue, this statistical optical process can be modeled by a mean optical path length (MOPL) that is traveled by theses photons in average through the tissue and which is considered as the length of the primary optical path.

The amount of attenuation of the IR-light depends on the length of this primary optical path. The longer the distance between source and detector, the longer the MOPL will be and the deeper the photons will penetrate into the tissue (in average), resulting in increased attenuation of the IR-light. By contrast, if the source-detector-separation (SDS), measured as a direct line-of-sight between source and detector, is small, the mean penetration depth (MPD) will also be small, the optical attenuation will be low, and the measured intensities will increase (for a given emitted intensity).

One severe drawback of such sensors is that they are sensitive on the location on which they are placed on the skin. For example, if a liver spot is present just below the primary detector, part of the IR-light can be absorbed by pigments present in the liver spot and this can have a detrimental effect on the accuracy and reliability of the optical measurement. This is a severe problem for example in live monitoring of vital parameters using sensors as described above, in particular, because relocation of the sensor on the skin cannot always be avoided.

Starting from this background, one object of the invention is to increase the accuracy and robustness of such optical measurements. It is therefore an object of the present invention to further develop methods and sensors as introduced at the beginning.

In accordance with the present invention, a measurement method is provided according to claim 1, which solves the afore-mentioned problem. In particular the invention proposes a method as introduced at the beginning, which, in addition, is characterized in that the method comprises the following additional steps: determining for each of the at least two measurement wavelengths (λₘ₁, λₘ₂) a wavelength specific correction factor (c₁(λₘ₁), c₂(λₘ₂)); calculating an estimate of the at least one optical or physiological parameter based on said measured primary intensities (Iₘ₁, Iₘ₂) and based on said at least two wavelength specific correction factors (c₁(λₘ₁), c₂(λₘ₂)).

This approach thus describes a multiwavelength-method for determining an optical or physiological parameter, such as a concentration of a particular chromophore in a tissue, wherein for each measurement wavelength, a suitable wavelength specific correction is applied to obtain a more accurate estimate of said parameter. In other words, it is suggested to correct measurement results, in particular measured intensities, obtained with the at least two measurement wavelengths individually, each time using a specific correction factor that is applicable to the particular wavelength. In conclusion, at least two different wavelength specific correction factors are determined and used for calculating a corrected and hence more accurate estimate of said at least one optical or physiological parameter. The medium can be a light scattering medium such as human or animal tissue, in particular nervous tissue.

The invention assumes that usual superficial absorbers (such as hairs, liver spots, bruises, or air gaps or the like), which can affect the measured intensity at the detector, show a wavelength dependence. For melanin, for example, it is known that the absorption curve shows a monotonous slope, with absorption being higher at lower wavelengths and decreasing at higher wavelengths. Based on the assumption that melanin is present as an absorber, as one possible example, a respective wavelength-dependent coupling factor k(λ) can be estimated/determined. By using several auxiliary wavelengths in a calibration measurement for determining said at least one wavelength specific correction factor, the respective wavelength response (resulting from light being backscattered by tissue for example) can be measured precisely and corresponding wavelength-dependent coupling factors k(λ) can be determined/calculated for each of the measurement wavelengths used. Such coupling factors k(λ) can then be used as correction factors in the meaning of the invention. With previous approaches, significant measurement errors occurred even in cases in which different coupling factors (which in reality depend on wavelength) of two detectors or two sources (in each case, used for measuring the primary radiation) were determined with a single auxiliary wavelength. This may be the case, for example, if the coupling of the measurement wavelength into or out of the medium is largely different between two measurement wavelengths used, as these wavelengths may penetrate the medium or leave the medium at in a different manner. For example, one wavelength may pass through a freckle almost unattenuated, while another may be strongly attenuated by the freckle.

One reason for such a difference can thus be an absorber (such as melanin) that is present in the optical path of a first measurement wavelength and in the optical path of a second measurement wavelength, but which attenuates the two measurement wavelengths differently, as the attenuation (absorption, scattering etc.) will be typically wavelength dependent (i.e. dispersive). In such a case, the intensity measured at the respective detector and corrected using a single common correction factor determined with only one auxiliary wavelength will in general be either overestimated or underestimated, at least for one of the two distinct measurement wavelengths.

In contrast, the invention offers the advantage that either by means of an estimation/calculation/interpolation or, preferably, by means of an actual calibration measurement, in particular using at least two, preferably at least three, different auxiliary wavelengths (which may be identical to or at least close to the measurement wavelengths used), accurate wavelength-dependent correction factors can be determined and the respective (wavelength specific) correction of the measured primary intensities can be performed more accurately for each of the used measurement wavelengths. In other words, it is possible to implement the method as a self-calibration algorithm in which each of the primary intensities measured for one of the measurement wavelengths employed is accurately and specifically corrected using a respective and specific correction factor that is tailored to / adapted to the respective measurement wavelength. As a result, the measured intensity values can all be optimally self-calibrated for each one of the measurement wavelengths.

Depending on the light source used for emitting the wavelengths, in particular when using LEDs (which do not show as sharp emission lines as lasers do), the two distinct wavelengths may be actually comprised within a limited emission spectrum, centered around a peak emission wavelength, which shows the maximum intensity within the emitted spectrum. Therefore, the term "distinct wavelength" may be understood in the sense that the two wavelengths are respective peak emission wavelengths of a respective spectrum. The emission spectra may thus overlap; however, it is preferable if the spectra do not overlap or if at least the respective Full Width-Half Maximum (FWHM) of the spectra do not overlap. The minimum distance between these two distinct peak emission wavelengths may be at least 10 nm or more. For example, the method can be used in a situation in which four different measurement wavelengths are employed such as: 690, 760, 805, 830 nm.

Light sources that may be employed can be LEDs, laser diodes, or end facets of light fibers. Suitable detectors are photodiodes, phototransistors, photomultipliers, CCDs, CMOS-imagers or other optoelectronic detectors. Such components may also be used in combination with light fibers, optical lenses and optical apertures, as is well known in the art.

The method presented so far can be further elaborated and implemented in various ways, which is described in the subclaims and in the following:
For example, according to a preferred embodiment, at least four, or event at least 6, distinct measurement wavelengths may be used. This way, a very robust and accurate optical measurement can be implemented.

For example, one embodiment suggests that the method further comprises the following steps: irradiating the medium with a secondary radiation comprising at least two distinct auxiliary wavelengths (λₐ₁ ≠ λₐ₂) which are emitted by at least one auxiliary light source (for example, there may be at least two different auxiliary light sources, each emitting a respective auxiliary wavelengths λₐᵢ); measuring secondary intensities (Iₛ₁, Iₛ₂) of the secondary radiation for each (i.e. , separately for each) of said at least two auxiliary wavelengths (λₐ₁, λₐ₂), after said secondary radiation has propagated through said medium along a respective secondary optical path; determining the wavelength specific correction factors (c₁(λₘ₁), c₂(λₘ₂)) based on said secondary intensities (Iₛ₁, Iₛ₂), which result from the auxiliary wavelengths (λₐ₁, λₐ₂) emitted by the at least one auxiliary light source.

We note at this point, that the secondary optical light paths which are traveled by the respective auxiliary wavelengths may be spatially distinct from each other.

This approach thus proposes to employ at least one auxiliary light source (preferably one auxiliary light source per auxiliary wavelengths used may be employed) to enable an additional calibration measurement of the secondary intensities, which are then used for calculating the desired at least two correction factors. In other words, it is proposed to send at least two distinct auxiliary wavelengths along two respective secondary optical paths through the medium, to detect this secondary radiation respectively, and to measure the resulting respective secondary intensities for each of the at least two auxiliary wavelengths separately. This approach allows to gain information on the wavelength dependence of the correction that needs to be applied to the primary measurement using the primary intensities.

Similar to the measurement wavelengths, the term "distinct" may be understood as explained previously; hence, each auxiliary wavelength can be a peak emission wavelength of a broader emission spectrum that is emitted by the respective auxiliary light source.

The method can be implemented, for example, in that a first estimate of the at least one optical or physiological parameter is determined based on said measured primary intensities. Using the wavelength specific correction factors, determined based on the secondary intensities (which were measured using the at least two auxiliary wavelengths), the first estimate may then be corrected/modified to yield a second corrected estimate. However, it is also possible to calculate the corrected estimate (as the finale estimate) directly based on the determined wavelength specific correction factors.

We note at this point, that in the typical application case of the method such as a near-infrared-spectroscopy(NIRS)-measurement in human tissue, this approach can compensate/correct for wavelength dependent effects, which arise from tissue areas which are penetrated by both the measurement wavelength to be corrected (to be more precise: the intensity measured for the respective measurement wavelength is to be corrected) and by the auxiliary wavelength that is employed for the correction. This even applies, if the auxiliary wavelength deviates from the measurement wavelength (this may be mitigated by interpolation, as will be explained below). It is important for efficient correction that the auxiliary wavelength and the measurement wavelength are probing the same tissue area, at least in part.

The measured primary and/or secondary intensities can be back-scattered intensities, which are measured after the respective radiation has been backscattered by said medium, or for example an intensity measured in transmission (for example when wrapping a sensor around an artery and sending light straight through the artery (in which case only forward scattered light will be measured).

In addition, the measured primary and/or secondary intensities can result from absorption and/or scattering and/or coupling factors, which all can diminish the measured intensities.

According to a preferred embodiment, at least three, or most preferably at least four, or even at least six, different/distinct auxiliary wavelengths (λa1 ≠ λa2 ≠ λa3) may be employed. In this case, the number of measurement wavelengths may be even smaller than the number of auxiliary wavelengths employed. Using more than two distinct auxiliary wavelengths can improve the accuracy in determining the correction factors, in particular when using a dispersion model and/or when interpolating the factors.

According to another embodiment, for each measurement wavelength employed for determining said parameter, a corresponding auxiliary wavelength may be employed, which may be emitted by a corresponding auxiliary light source. In such a case, it is most preferably if all of the auxiliary wavelengths are identical to or closely match (wavelength difference of < 10 nm) the corresponding measurement wavelength.

According to yet another preferred embodiment, the at least two distinct auxiliary wavelengths may be emitted by at least two auxiliary light sources. Preferably, these auxiliary light sources may be operable independently from each other, in particular such that a time-multiplexing approach can be used for emitting the auxiliary wavelengths at different points in time; this way, the two auxiliary wavelengths can be detected using a single detector, which may be a primary detector or an auxiliary detector.

Another preferred embodiment of the method employs ten different/distinct measurement wavelengths and four different auxiliary wavelengths. Preferably, the auxiliary wavelength should be distributed evenly (at least approximately) within the spectrum range defined by the measurement wavelengths.

Another implementation of the method proposes - alternatively or additionally to using an auxiliary light source - to employ at least one auxiliary detector to enable a calibration measurement of secondary intensities, which are required for calculating the desired at least two correction factors. Accordingly, the method may further comprises the following steps (in particular additionally to using auxiliary light sources): detecting a secondary radiation comprising the at least two measurement wavelengths with at least one auxiliary detector, wherein the secondary radiation has traveled along a secondary optical path that is (at least partially) different from the primary optical path along which said primary radiation has traveled; measuring secondary intensities of the secondary radiation using the at least one auxiliary detector for each of said at least two measurement wavelengths after said secondary radiation has propagated through said medium along the secondary optical path; determining the wavelength specific correction factors based on said secondary intensities, which have been measured with the at least one auxiliary detector;

In this particular case, the measurement wavelengths comprised in the secondary radiation (which may be emitted by a primary light source) and having traveled said secondary optical path can be considered as auxiliary wavelengths. The primary and secondary intensities will be different however, because they are measured with a primary detector and an auxiliary detector, respectively, and these detectors can be located at different distances from the primary light source which emitted the light that is detected by the respective detector, thus resulting in different optical paths.

We note at this point, that the general correction method proposed herein can thus be used no matter if auxiliary light sources or auxiliary detectors are employed, because in principle, the path of light can simply be inverted by swapping the respective positions of sources and detectors, which will become evident from the examples shown in the figures.

We also note, that at least one of the at least two distinct auxiliary wavelengths, in particular all of the mentioned auxiliary wavelengths, may be either identical to (λₐ₁ = λₘ₁ and/or λₐ₂ = λₘ₂) or distinct from (λₐ₁ ≠ λₘ₁ and/or λₐ₂ ≠ λₘ₂) a respective one of the at least two measurement wavelengths (λₘ₁, λₘ₂).

In case the auxiliary wavelengths are identical or at least very close (< 25 nm distance) to the measurement wavelengths, it is preferable, if the relative emission spectra of the light sources used for emitting these wavelengths are comparable to each other in the bandwidth (less than 50 nm difference in bandwidth). In case the auxiliary wavelengths are remote from (> 50 nm distance) the measurement wavelengths, it is preferable if the light sources used for emitting these wavelengths each show narrow emission spectra (FWHM < 100 nm).

In case there is a significant distance between a measurement wavelength and its corresponding auxiliary wavelength, it is preferable, if each of said at least two auxiliary wavelengths varies, respectively, by less than 30% from a corresponding one of the at least two measurement wavelengths. Preferably, said variation may by less than 10%, and most preferably even less than 3%.

Also note that even when one particular auxiliary wavelength does not perfectly match one of the used measurement wavelengths, it is still possible to at least accurately approximate / calculate a correction factor that is specific for said particular measurement wavelength. For example, when using at least two distinct auxiliary wavelengths, it is possible to adapt a model of the correction factor that allows interpolation to almost any measurement wavelength; of course, the accuracy of the correction will be higher, the closer the auxiliary wavelength matches a particular measurement wavelength and the more reasonable the model can reflect the wavelength dependence of the underlying mechanism affecting the measurement. For example, linear interpolation will not work well, if the wavelength dependent phenomenon to be corrected shows abrupt changes with wavelength. However, within a limited wavelength band of measurement wavelength, a linear model may lead to an accurate estimation of the necessary correction factors to be applied.

Accordingly, at least one of the wavelength specific correction factors may be interpolated mathematically, based on the measured secondary intensities. In such a case, the secondary intensities can thus be measured without using the measurement wavelength, for which the corresponding correction factor is interpolated. Using this approach, it is possible to correct the measurement values of primary intensities which are measured with a measurement wavelength that is not comprised in the used auxiliary wavelengths. For example, if peak emission wavelengths of 530 nm and 780 nm, respectively, are used as auxiliary wavelengths, the secondary intensities measured at 530 nm and at 780 nm may be used to compute correction factors applicable to measurement wavelengths which lie in between 530 nm and 780 nm, or beyond 780 nm, or below 530 nm. This computing can be based on a model describing the dispersion (= wavelength dependent change) of the correction factors, as will now be explained in more detail.

According to one particular embodiment, based on the at least two different secondary intensities, which are each measured for one of said two distinct auxiliary wavelengths, respectively, a (pre-chosen) model c(λ) describing the wavelength dependence of at least one correction factor c(λ), which affects said measured primary intensities, is adapted. For example, if the chosen model is a linear model, the slope of the linear relation may be adapted based on the measured secondary intensities. In case of a more sophisticated model, a best-fit-approximation may be used to adapt multiple parameters of that sophisticated model.

Such an adapted model may then be used to determine said estimate of the parameter to be determined with the optical sensor. In such a case, the at least two wavelength specific correction factors can thus be calculated from said adapted model c(λ), i.e., for each of said at least two measurement wavelengths λₘᵢ a respective correction factor cᵢ(λₘᵢ) can be determined based on the adapted model. We note, that at least one of said at least two auxiliary wavelengths (or even all of them) may be distinct from and thus not match any of said at least two measurement wavelengths; thanks to the interpolation, a matching of all auxiliary wavelengths with one of the measurement wavelengths is no impediment for accurately correcting for wavelength dependent phenomena.

Depending on the implementation of the method, the at least two wavelength specific correction factors can be understood as defining a respective wavelength specific correction that is to be applied to the primary intensities measured for each of said at least two measurement wavelengths.

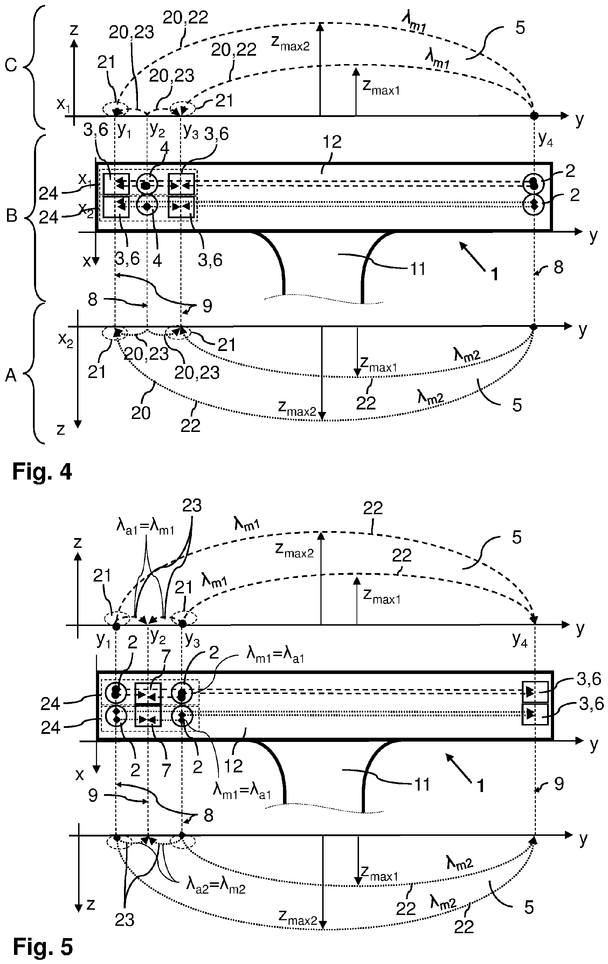

For example, the respective correction factor may be simply a ratio of two secondary intensities I_{s1, SDSI} (λₐ₁) /I_{s1,SDS2} (λₐ₁) . These two secondary intensities may be measured at different locations (using different detectors) and/or result from light emitted at different locations into the tissue (e.g., using different light sources). The two secondary intensities may thus be measured using the same auxiliary wavelength λₐ₁; however, these two intensities may have been measured either using the same (primary) detectors, with which the primary intensities (to be corrected) have been measured; or (in case separate auxiliary detectors are used - see Figure 5) they may have been measured using the same primary light sources (in this case the respective auxiliary wavelengths and the respective measurement wavelength will be identical: λₐᵢ = λₘᵢ).

For highlighting possible applications of the method, it is emphasized here that the at least two wavelength specific correction factors may correct the calculated estimate of said parameter for example with respect to: a wavelength dependent coupling factor k(λ), in which case the coupling factor k(λ) may define a loss of light (e.g., due to scattering or optical reflection) occurring at a specific wavelength at an interface of said medium, for example; and/or an absorption or scattering spectrum inside the medium (this spectrum being wavelength dependent) - note that such a compensation requires at least some overlap of the optical paths traveled by the respective auxiliary and measurement wavelength (such that they probe at least partly the same area of the medium); and/or an optical obstruction which shows a wavelength dependent transmission or scattering spectrum (i.e. the scattering coefficient is wavelength dependent). Such an obstruction in the path of the respective measurement wavelength may be a hair, a pimple, a blemish or a pigmentation mark on the skin which is optically probed with a sensor implementing the method; it may even be a contamination on the sensor itself. We note that the correction will, of course, only be meaningful, if the obstruction is also optically probed by the auxiliary wavelength, because otherwise, the secondary intensity measured with that auxiliary wavelength will not alter due to the presence of the obstruction.

Such corrections are made possible, in particular, if the optical paths - which the auxiliary wavelength and the corresponding measurement wavelength (which is corrected using said auxiliary wavelength) take, respectively, during the respective measurements of the primary and secondary intensities - show at least a partial overlap. In more detail, the described wavelength dependent phenomena (optical coupling/absorption & scattering/obstructions) can be compensated, if the overlap is located in an area (for example the skin interface just beneath a detector used in both measurements) in which the phenomenon occurs or is located (in case of an obstruction). Of course, any movement of the sensor between measuring the secondary and primary intensities must be avoided, because otherwise, the overlap may be lost or significantly reduced. For example, if the secondary intensities are measured at the location of a liver spot, then the sensor is moved, and afterwards the primary intensities are measured at a different location where there is no such liver spot, the correction factors will not be valid and the estimate will be adulterated and hence inaccurate. Therefore, it is generally recommendable to measure the primary and secondary intensities within a short time interval of a few msec.

The estimate of the parameter may be determined/calculated based on at least one ratio of the primary intensities, which have been measured for each of said at least two measurement wavelengths. Accordingly, each measured primary intensity may be corrected using one of said wavelength specific correction factors.

The auxiliary light source may emit a wavelength spectrum that is broader than a respective spectrum of said at least one primary light source. Accordingly, an auxiliary detector may be able to detect a broader wavelength spectrum than one of the primary detectors used.

For the primary light source(s), it is preferably if their respective emission spectrum is relatively narrow, for example with a FWHM-width of less than 100 nm. Using a broader emission spectrum in the auxiliary light source is possible. This way, some wavelength averaging can be obtained, as all wavelengths emitted by the auxiliary light source can be detected simultaneously with one single detector (e.g. a photodiode that is not wavelength selective). As a result, the auxiliary wavelengths will provide an (weighted) average correction factor for the wavelength range that is covered by the emission spectrum of the auxiliary light source, which may be, for example, a green LED that can also be employed for additional pulse oximetry measurements.

However, the wavelength correction factor will be determined most accurately if the auxiliary light source also shows a narrow emission spectrum (e.g., FWHM < 100 nm) and its peak emission wavelength is within close range (< 25 nm) of the peak wavelength of the primary light source whose measurement wavelength is to be corrected. This should apply to all auxiliary wavelengths employed and will be in particular the case, when using an auxiliary detector, because in this case, the auxiliary wavelength and the measurement wavelength can be emitted by the same primary light source and can thus be identical.

One embodiment suggests that for each of the at least two measurement wavelengths, at least two different primary intensities are measured using two different source-detector-separations, respectively. In this case, the estimate of the parameter may be calculated from respective ratios of said at least two different primary intensities. In other words, it is preferable if the estimate is based on at least two such ratios. Even more preferably, at least two such estimates may be calculated to provide redundancy and a higher accuracy to the optical measurement.

By using two measuring distances (i.e., two source-detector-separations (SDS1, SDS2)) of different lengths, it is possible to define different mean optical path lengths (OPL) in said medium and as a result different penetration depth. Using this approach, deeper tissue layers ("deep tissue") may be measured and may be distinguished from shallow tissue layers. The respective concentrations C of chromophores present in the tissue can then be determined, for example, by comparing the intensities determined in at least two distinct detector locations D1 and D2. This may be done by using said ratios of the at least two different primary intensities, which can be measured for each one of the at the at least two measurement wavelengths, respectively. Source-detector-separations (SDS) may be the distances (measured as a direct line of sight) between a light source and an associated detector. Hence two detectors located at different locations and hence different distances from a common emitting location / common source are sufficient for defining two different SDS. Alternatively, two sources located at different locations and one common detector located at a common detecting location can also define two different SDS.

According to one aspect of the method, the same two different source-detector-separations (SDS1ₘ₁=SDS1ₘ₂; SDS2ₘ₁=SDS2ₘ₂) may be used for measuring said at least two different primary intensities (Iₘ₁₁,Iₘ₁₂; Iₘ₂₁,Iₘ₂₂) for each of the measurement wavelengths. This may be done, in particular using, at least two primary light sources located at a common emission location and two primary detectors located at two distinct source-detector-separations (SDS1ₘ₁=SDS1ₘ₂ ≠ SDS2ₘ₁=SDS2ₘ₂) from said common emission location.

Most preferably is the use of a layout, in which one of the at least one auxiliary light sources is located equidistant from two primary detectors. This allows to calculate two wavelength and location specific correction factors c₁₁(λₘ₁,y₁) and c₁₃(λₘ₁,y₃) which can compensate optical phenomena occurring at different locations y1, y3 for the measurement wavelength λₘ₁ to be corrected. Such a design may be understood as a symmetric optical calibration set (OCS). These two correction factors can thus be used for correcting primary intensities which are measured with λₘ₁ using the mentioned two primary detectors. Preferably such a equidistant layout may be used for all of the auxiliary light sources employed.

The layout of the sensor used for implementing the method can be such that for an ideal homogenous medium, the determined at least two wavelength specific correction factors (c1(λm1), c2(λm2)) would be equal.

However, it is also possible that the at least one auxiliary light source(s) is/are located non-equidistant from the two primary detectors (at known distances from the detectors). The latter alternative means that the auxiliary light source may be located closer to one of the two primary detectors as to the other of the two primary detectors. Such an asymmetric OCS can still be used for calculating wavelength specific correction factors.

As already explained, at least one primary detector may be employed for measuring said primary intensities. In this case, the at least one auxiliary light source may be located closer to said at least one primary detector than to said at least one primary light source. Such a design allows that a maximum source-detector-separation (SDS), which is measurable with the auxiliary light source, is smaller than a maximum SDS measurable with said at least one primary light source. This approach results in a highly compact design, yet still allows correction at sufficient accuracy. Preferably, the at least one auxiliary light source may be located at least three times or even five times closer to said at least one primary detector than said at least one primary light source. First of all, such compact designs allow detection of even small variations in the coupling efficiencies below the primary detector. Secondly, when using a rigid PCB, a smaller rigid area is needed on which a primary detector, an auxiliary source, and possibly an auxiliary detector can be placed precisely next to each other. If this rigid area can be smaller, then the sensor as a whole will be more flexible, which is beneficial for achieving a good skin contact. Thirdly, the light intensities emitted by the primary source will differ less as detected by a primary detector and by an auxiliary detector used. A possible drawback is, however, that the auxiliary detector must then be larger as compared to a case in which the auxiliary detector is closer to a corresponding primary source. Fourthly, the correction for variations in optical coupling and superficial inhomogeneities (which affect the coupling) are mainly determined by the more superficial tissue layers (i.e., by light paths with shallow penetration depths). Accordingly, the precision of the optical measurement will be higher, as the (multi-wavelength) correction will be more precise.

In conclusion, the at least one auxiliary wavelength(s) may thus be emitted from an auxiliary point of entry which is distant from at least one primary point of entry from which said at least two measurement wavelengths are emitted. In other words, said at least one auxiliary light source may be located at a minimum distance from said at least one primary light source. This minimum distance may be larger than a smallest SDS that is measurable with said at least one primary light source.

The primary intensities can be detected at one, preferably at two, distinct common detection location(s). In such a case, two different SDS can be measurable for each of the at least two distinct measurement wavelengths. It is further preferable, if the two common detection locations are located at respective distances SDS1, SDS2 from a common emission location from which said at least two measurement wavelengths are emitted by said at least one primary light source. Most preferably, the at least one auxiliary light source can then be located in between one of said two common detection locations and the common emission location. This approach can result in a symmetric OCS, as described above.

The primary intensities can also be measured using at least one primary detector while the secondary intensities are measured with at least one auxiliary detector. This approach is applicable, for example if no auxiliary light sources are used.

Alternatively (or additionally), the primary intensities and the secondary intensities can also be both measured using at least one common detector, which may be a primary detector. This approach is particularly applicable, if no auxiliary detector but at least one auxiliary light source is used.

To mitigate the effect of movements of the sensor and to further improve the reliability, the method may be further elaborated in in that each time an updated value is determined for the estimate, a calibration measurement is performed beforehand using the secondary radiation to determine updated values of the at least two correction factors. Even more preferably, a multitude of such calibration measurements may be performed to obtain (updated) average values for said at least two correction factors prior to determining an updated value of said secondary estimate.

It is also possible to perform a calibration measurement using the secondary radiation to calculate updated values of the at least two correction factors, as soon as a movement of an optical sensor, which is used for determining said estimate / for measuring the secondary radiation, is detected. This detection may be achieved automatically using built-in sensors (e.g. gyros, accelerometers and the like or an optical detector) of the optical sensor. Accordingly, the optical sensor may be configured to detect a movement using at least one built-in sensor. An electronic unit of the optical sensor may be configured to trigger such a calibration measurement, in reaction to a movement detected with the built-in sensor.

In other words, the movement (which may be a movement of the optical sensor itself or of the object optically probed with the optical sensor) is detected by the sensor itself, for example based on the read-out of an accelerometer or of an optical detector or any other suitable built-in sensor.

In accordance with the present invention, there is also provided an optical sensor, which solves the afore-mentioned problem. In particular, there is provided an optical sensor as introduced at the beginning, which is further characterized in that the sensor features at least one auxiliary light source for emitting a secondary radiation comprising at least two distinct auxiliary wavelengths (λₐ₁ ≠ λₐ₂) .

Alternatively, or additionally, the sensor may also comprise at least one auxiliary detector which is capable of measuring secondary intensities of a secondary radiation comprising the at least two distinct measurement wavelengths. In this case, the measurement of the secondary intensities occurs after said secondary radiation has propagated through said medium along a secondary optical path which is different from said primary optical path.

Both approaches, if used alone or in combination, can be best exploited if the electronic unit of the optical sensor is configured to implement a method according to one of the claims directed towards a method and/or as described herein. The electronic unit can thus finally output said estimate of the at least one optical or physiological parameter, for example by displaying it as a live measurement value on a display.

Of course, it is of particular advantage, if the method explained in detail above is implemented by the optical sensor just described. However, the optical sensor may also be distinguished from prior art sensors based on the specific layout of sources and detectors described before with respect to the method. For example, as described before, the at least one auxiliary light source may be located at a distance from said at least one primary light source. Similarly, the at least one auxiliary detector may be located at a distance from said at least one primary detector.

The estimate determined by the electronic unit may be outputted as an analogue or digital signal or it may be outputted and/or visualized using a display or other electronic output device. It is also evident, that this sensor may have components arranged and configured as described before with respect to the method according to the invention. Thus, this sensor may be equipped and configured to implement any of the embodiments of the method explained before or as claimed in the claims directed towards a method.

We also note at this point, that the two distinct auxiliary wavelengths can also be comprised in a broadband emission spectrum of a single auxiliary light source. This is because by using (spectral) optical filters and/or time-multiplexing approaches, each of the two distinct auxiliary wavelengths can still be detected individually/separately to measure two separate secondary intensities. These secondary intensities can then be used for determining at least two (different) wavelength specific correction factors. Therefore, a single auxiliary light source may be sufficient to implement the method according to the invention.

Examples of the present invention will now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying drawings, where features with corresponding technical function are referenced with same numerals even when these features differ in shape or design:
- Fig. 1: is a partial cross-sectional side view of an optical sensor according to the invention, which illuminates a tissue medium with infrared light,
- Fig. 2: shows a perspective view of inner components of the sensor of Figure 1,
- Fig. 3: illustrates a schematical representation of the sensor of Figures 1 and 2, with the view directed onto the lower contact surface of the sensor, as seen from below,
- Fig. 4: shows a more detailed schematical representation of the sensor of Figures 1 and 2, again with the view directed onto the lower contact surface of the sensor and also with illustrations of the mean optical paths of photons emitted by the sensor in the tissue,
- Fig. 5: shows a similar schematic as Figure 4, but for a different sensor design according to the invention,
- Fig. 6: shows a similar schematic as Figure 4, but for a yet another different sensor design according to the invention,
- Fig. 7: presents a more realistic possible implementation of an optical sensor with an optical layout of sources and detectors according to the invention,
- Fig. 8: illustrates how wavelength specific correction factors cᵢ(λₘᵢ) can be derived by interpolation using at least two distinct auxiliary wavelengths and,
- Fig. 9: illustrates the case when the respective auxiliary wavelength matches a corresponding measurement wavelength.

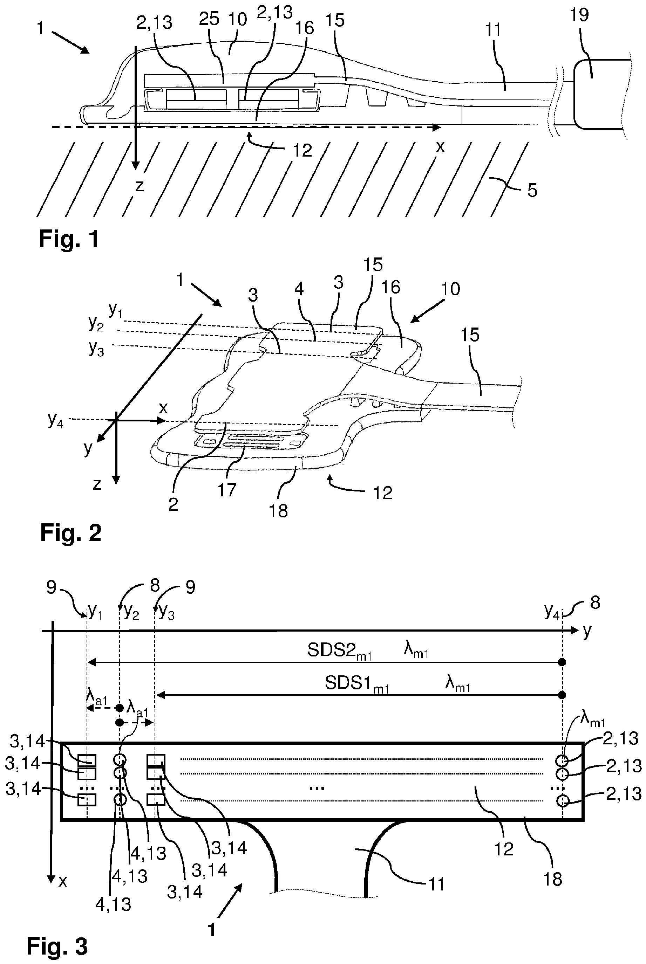

Figure 1 is a partial cross-sectional side view of an optical sensor 1 according to the invention. The sensor 1 illuminates a tissue medium 5 with infrared (IR) light which is emitted by several LEDs 13 and detected with several photodiodes 14, after the radiation has traveled through the medium 5 by random scattering processes. These electronic components 13, 14 are mounted on a stiff printed-circuit-board (PCB) 25 located in the head 10 of the optical sensor 1, the PCB 25 being electrically connected to an electronic unit 19 via a flat cable 11 that is running along a longitudinal x-direction of the optical sensor 1. The electronic unit 19 controls the operation of the LEDs 13 and photodiodes 14 and comprises a computing unit for computing an estimate of a local concentration of oxy- and deoxyhemoglobin (as two physiological parameters) in the tissue 5, based on primary intensities Iₘᵢ which are measured with the photodiodes 14 and which depend on the attenuation of the IR-radiation in the tissue 5.

Figure 2 shows more details of inner components of the sensor head 10: During a measurement, the sensor head 10 is brought into skin contact with its contact surface 12 that is formed by the lower side of a lower member 16 of the head 10. The IR-light is emitted by the LEDs 13 along the z-direction through respective slit apertures 17, such that the respective point of entry of the light into the tissue is well defined in the transverse y-direction.

Figure 3 illustrates a schematical representation of the sensor head 10 of Figures 1 and 2, with the view directed onto the lower contact surface 12 of the sensor 1, as seen from below. By comparing this schematic layout with the perspective view of the sensor head 10 of Figure 2, it can be seen that the optical sensor 1 features a number on N primary light sources 2 which are arranged along the x-axis at a common emission location 8 with y-coordinate y₄ (on the far right of Figure 3). Each of these N primary light sources 2 emits a respective measurement wavelengths λₘ₁ .. λ_{mN}. Each of these N measurement wavelengths λₘ₁ .. λ_{mN} can be detected, respectively, by two dedicated respective primary detectors 3, which are formed by respective photodiodes 14 and measure the respective IR-light after its propagation through the tissue along a respective primary optical path 22 (cf. Figure 4). In other words, each horizontal line in the schematic of Figure 3 is dedicated to measuring one of the N measurement wavelengths. In more detail, each measurement wavelength can be evaluated with respective detectors 3, which are located at distances SDS1 and SDS2, respectively, from the corresponding primary source 2. This is indicated exemplarily by arrows for the first measurement wavelengths λₘ₁. For this purpose, the respective two primary detectors 3, which are used for evaluating a respective one of the N measurement wavelengths, are located in common detection locations 9 at y-coordinates yi and y₃, respectively.

As visible in Figure 3, for each of the N measurement wavelengths λₘ₁ .. λ_{mN} a respective auxiliary light source 4 is placed in between the respective two primary detectors 3; hence a total of N auxiliary light sources 4 are employed, one for each measurement wavelength. In more detail, the auxiliary light sources 4 are placed equidistant from the respective two primary detectors 3 (located at yi and y₃), respectively, such that these auxiliary sources 4 are also located on a common emission location 8 at y-coordinate y₂. Each auxiliary light source 4 emits an auxiliary wavelength λₐᵢ that is identical to or at least very close to (deviation of less than 10 nm) the respective measurement wavelength λₘᵢ (λₐᵢ≈λₘᵢ).

The measurement concept proposed herein, and which is implemented by the electronic unit 19 of the optical sensor 1, will now become evident with a look onto Figure 4: Figure 4 provides a more detailed schematical representation of the sensor of Figures 1 to 3, again with the view directed onto the lower contact surface 12 (i.e., in negative z-direction). Figure 4 also provides illustrations of the mean optical paths 20 which the photons emitted by the sources 2, 4, travel through the tissue 5 in the yz-plane at x-coordinates x₁ (upper graph) and x₂ (lower graph), exemplarily for two measurement wavelength λₘ₁(dashed curves 20/upper yz-graph) and λₘ₂(dotted curves 20/lower yz-graph): As the optical scattering occurring in the tissue 5 is a statistical process, a single photon will follow a complex zig-zag-course through the medium 5 (not shown). Some photons will propagate in more shallow regions of the tissue 5, while others will penetrate deeper into the tissue 5. Of course, there are also a lot of photons which are lost and never reach the respective detector 3. However, all photons reaching the respective detector 3 can be modeled by the illustrated banana-shaped curves 20, which represent the mean optical paths 20 traveled by these photons.

Accordingly, in the case of Figure 4, the optical sensor can measure at least four different primary intensities: I₁ = Iₘ₁ (λₘ₁, y₁), I₂ = Iₘ₁(λₘ₁, y₃), I₃ = Iₘ₂(λₘ₂, y₁), I₄ = Iₘ₂(λₘ₂, y₃). Furthermore, two source-detector-separation (SDS) are used (SDS1 = y₄-y₁ and SDS2 = y₄-y₃) in these measurements, resulting in two different mean optical penetration depths (zₘₐₓ₁ and zₘₐₓ₂) that can be optically probed.

It also evident from the curves 20 and the illustrated (average) maximum penetration depth zₘₐₓᵢ in Figure 4, that the larger the source-detector-separation (SDS) between a source 2 and allocated detector 3 used for measuring the radiation sent out by this source 2, the deeper the tissue regions will be that is probed by that primary radiation. Note that due to the relative short distances between the auxiliary sources 4 and detectors 3 (which are five times smaller than the smallest SDS 1 used for the primary radiation) the auxiliary light that is sent out by these sources 4 only travels a very short distance through the tissue along the illustrated secondary optical paths 23 and is thus mainly unaffected by optical variations occurring deeper in the tissue.

In fact, the SDS between the auxiliary light source 4 and the neighboring primary detectors 6 is less than 5 mm in Figure 4. By contrast, SDS1 and SDS2, which are used for the primary radiation, are much longer, in fact more than 25 mm long.

As the same detectors 3 are used for measuring the auxiliary light comprising the respective auxiliary wavelength λₐᵢ, there is an overlap 21 visible in Figure 4 between the primary optical paths 22 traveled (in average) by the primary radiation comprised of the respective measurement wavelength λₘᵢ and the respective secondary optical paths 23 traveled by secondary radiation comprised of the respective auxiliary wavelength λₐᵢ and emitted by the respective auxiliary light source 4. Note that the light from one of the auxiliary light source 4 forms two secondary optical paths 23 (to the left and to the right of the source 4), respectively, as the respective auxiliary wavelength λₐᵢ is picked up by the respective detector 3 positioned at y-coordinate y₁ and by the detector 3 positioned at y-coordinate y₃ (cf. also Figure 3).

If a liver spot, as one example of a possible optical obstruction, is present at the skin interface of the tissue 5 at the y-location y₃ in Figure 4, for example, the calibration measurement that is performed by irradiating the medium 5 with the secondary radiation emitted by the respective auxiliary light source 4 and measuring a secondary intensity Iₛᵢ(yᵢ) of the secondary radiation for the respective auxiliary wavelength λₐᵢ with one of the detectors 3 will reveal, if this liver spot affects the measurement of the corresponding measurement wavelength λₘᵢ. For example, the liver spot may attenuate one of the measurement wavelengths λₘ₁ strongly, while transmitting other measurement wavelength λₘᵢ almost unchanged. In this case, the corresponding secondary intensity Iₛ₁(y₃) measured for the corresponding auxiliary wavelength λₐ₁ that is close to or even identical to that particular measurement wavelengths λₘ₁ and which is measured at location y₃ will be diminished. However, the secondary intensity Iₛ₁(y₁) measured at y-location y₁ may still be unaffected, if the liver spot does not extend to that location.

Based on the measured secondary intensities Iₛᵢ, the computing unit of the optical sensor 1 can thus compute for each of the at least two measurement wavelengths λₘ₁, λₘ₂ (provided by the optical sensor 1) a wavelength specific correction factor (c₁(λₘ₁, SDSj), c₂(λₘ₂, SDSj), ...) . As indicated, a wavelength specific correction factor may be computed for each of the source detector separations (SDS1, SDS2, etc.) employed for measuring the primary intensities Iₘᵢ(λₘᵢ, SDSⱼ). In other words, for each primary intensity measured for a specific wavelength and a specific SDS, a corresponding secondary intensity Iₛᵢ may be measured, using either a primary light source 2 or a primary detector 3 that defines the respective SDS.

For example, in Figure 4, the secondary intensities Iₛᵢ(λₐᵢ, SDSⱼ)are measured with the detectors 3, that are also employed for measuring the corresponding measurement wavelength / the primary intensities. Hence, in the example of Figure 4, the secondary intensities Iₛᵢ are measured using the same primary detectors 6 with which the primary intensities Iₘᵢ have been measured.

In Figure 5, by contrast, the secondary intensities Iₛᵢ(λₘᵢ, SDSⱼ)are measured using the same primary light sources 2, with which the primary intensities Iₘᵢ(λₘᵢ, SDSj) were measured; however, the secondary intensities are measured with separate auxiliary detectors 7.

Accordingly, the estimate, which the computing unit calculates for the concentrations of oxy- and deoxyhemoglobin, will be more accurate, because this calculation can consider a required correction of the primary intensities Iₘᵢ(λₘᵢ, SDSj) based on said wavelength specific correction factors cᵢ(λₘᵢ, SDSj). In more detail, this correction can compensate for any wavelength dependent optical variation that is occurring within the overlap region 21, which - in the example of Figure 4 - corresponds to a region where the IR-light leaves the tissue 5.

Further note that in Figure 4, the auxiliary light sources 4 are (each) located closer to the respective neighboring primary detector 6 than to the respective primary light source 2. In other words, each auxiliary light source 4 is located, respectively, at a minimum distance from said at least one primary light source 2, which is larger than a smallest source-detector-separation (namely SDS1, i.e., the y-distance between y₃ and y₄ - cf. Figure 3) that is measurable with the respective primary light source 2.

We also note with respect to Figure 4, that the primary intensities Iₘ₁ and Iₘ₂ are detected at two distinct common detection locations 9, namely by the respective primary detectors 6 located at y₁ and y₃. As visible in Figure 4, the respective auxiliary light source 4 is located in between said two common detection locations 9.

Based on Figure 4, we can also imagine how, according to the invention, interpolation can be used to calculate more than two wavelength specific correction factors cᵢ(λₘᵢ) for more than two measurement wavelengths λₘ₁, using only two auxiliary wavelengths λₐᵢ which are emitted by (one or) two respective auxiliary sources 4: For this purpose imagine to add two more primary sources 2 emitting a third and fourth distinct measurement wavelength λₘ₃ and λₘ₄ at location y₄ in Figure 4. First, these additional two primary sources 2 could be used together with the existing primary detectors 6 located at y₁ and y₃ to from two SDS, each for λₘ₃ and λₘ₄. In addition, the secondary intensities Iₛ₁(yᵢ) and Iₛ₂(yᵢ) measured for the two auxiliary wavelength λₐ₁ and λₐ₁ (emitted by the two auxiliary sources 4 shown in Figure 4), respectively, can then be used to establish (i.e., in particular to parameterize) a model c(λ) describing the wavelength dependent attenuation of the radiation by an optical obstruction (e.g., a liver spot, a contamination on the contact surface 12 or any other dispersive phenomenon affecting the propagation of the radiation). Based on the model and using techniques of interpolation, wavelength specific correction factors c₃(λₘ₃) and c₄(λₘ₄) for the measurement wavelengths λₘ₃ and λₘ₄ can then be computed/estimated, and these factors can be used to calculate more accurate estimates of the physiological parameters of interest, based on primary intensities Iₘ₃ and Iₘ₄, which have been measured with primary radiation of wavelengths λₘ₃ and λₘ₄ emitted by the new primary sources 2. Note that in such a case, the two distinct auxiliary wavelengths λₐ₁ and λₐ₂ can (each) be distinct from λₘ₃ and λₘ₄. Also note that in this case, the secondary intensities Iₛ₁, Iₛ₂ will be measured without using the measurement wavelength λₘ₃ and λₘ₄, for which the respective correction factor c₃(λₘ₃), c₄(λₘ₄) is interpolated.

Figure 5 provides more insights into details of the method and possible sensor layouts according to the invention as proposed herein: shown is a schematic analogous to that of Figure 4 with the difference that the sources 2, 4 and detectors 3 have been swapped. This is possible as the optical path of a photon can be inversed, in principle. Accordingly, the sensor layout of Figure 5 provides the same two different source-detector-separations SDS1 and SDS2 as the layout of Figure 4, however, the photons are now emitted by a set of two primary sources 2 located at y-locations y₁ and y₃, respectively, per measurement wavelengths λₘ₁, and the photons travel from these sources 2 along the illustrated respective primary optical paths 22 (from left to right in Figure 5) to a respective (single) primary detector 6 at y-location y₄.

To enable a calibration measurement for each measurement wavelengths λₘ₁, the respective auxiliary sources 4 have been replaced by respective auxiliary detectors 7 in the layout of Figure 5. The latter serve to measure secondary intensities Iₛᵢ, which result from respective secondary radiation that has traveled along the illustrated secondary optical paths 23 from the respective source 2 (at y-locations y₁ or y₂) to the respective auxiliary detector 7 at y-location y₂. For example, for measuring the secondary intensities Iₛ₁(y₃) at location y₃ and the primary intensity Iₘ₁(y₄) at location y₄, it is sufficient to drive the primary light source 2 at location y₃ thereby emitting λₘ₁ into the tissue 5 and measuring the resulting intensities with the detectors 6,7 at locations y₂ and y₄.

Also note that in Figure 5, the auxiliary detectors 7 are (each) located closer to the respective neighboring primary source 2 than to the respective primary detector 6.

Since the secondary intensities need to be measured for each wavelength individually, the auxiliary detectors 7 may have optical filters, which transmit only one or some of the wavelengths employed. Alternatively, sources 2 and detectors 7 may be controlled in a time-multiplexing scheme, to differentiate between different wavelengths (which are then emitted at different points in time).

In the example of Figure 5, the secondary radiations detected by the respective auxiliary detector 7, after the respective secondary radiation has traveled/propagated through the tissue 5 (in average) along the respective secondary optical path 23, can thus be considered as an auxiliary light, as this light (other than the light detected at location y₄) is not employed to measure the two physiological parameters of interest directly, but only serves to compute wavelength selective correction factors cᵢ(λₘᵢ), as previously explained. Note again, that the secondary optical paths 23 only optically probe superficial tissue, but do not penetrate deeply into the tissue 5, as the primary optical paths 22 do. As in this particular case, the auxiliary light is emitted by the same primary light sources 2, which also emit the photons forming the primary radiation, the auxiliary wavelengths are thus perfectly identical to the measurement wavelengths (λₐᵢ = λₘᵢ). Also note that in Figure 5 the secondary intensities Iₛᵢ are measured at the same y-location (namely using the respective auxiliary detector 7 located at y₂) but result from light emitted at different locations into the tissue (namely by the primary light sources 2 located at y₁ and y₃).

As indicated by the dashed ellipses in Figure 5, there are again overlaps 21 formed between the primary optical paths 22 and the secondary paths 23 just described such that the measured secondary intensities Iₛ₁ employed can provide a meaningful information about wavelength dependent phenomena occurring in these regions. Note, however, that (other than in Figure 4), these regions are now regions in which the IR-light enters the tissue 5.

Also note that in Figure 5, the primary intensities Iₘ₁, Iₘ₂ are measured using at least one primary detector 6 (located at y₄), while the secondary intensities Iₛ₁, Iₛ₂ are measured with at least one auxiliary detector 7 (located at y₂). In both cases, light emitted form primary sources 2 (located at y₁ and y₃) is used for these measurements, respectively. We note again, however, that the optical paths 22 and 23, employed in these measurements, are different. Hence primary and secondary radiations are measured, respectively.

In both Figures 4 and 5, for each measurement wavelength employed, a respective optical calibration set (OCS) 24 is thus formed either by a set of two primary detectors 6, with an auxiliary light source 4 placed midway between/equidistant from those detectors 6 (as in Figure 4); or by a set of two primary sources 2, with an auxiliary detector 7 placed midway between/equidistant from those two sources 2 (as in Figure 5). Of course, due to the principal exchangeability of sources 2 and detectors 3, the concepts illustrated in Figure 4 and Figure 5 can also be used in combination / can be mixed in a sensor layout according to the invention.

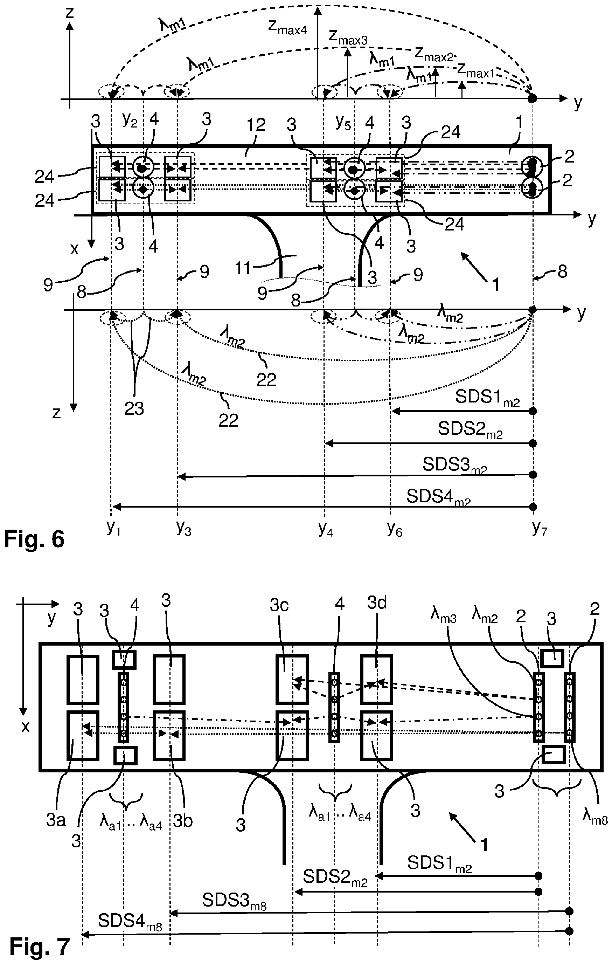

Figure 6 presents another possible implementation of an optical sensor 1 according to the invention. This sensor design is based on the layout of Figure 4 but introduces one additional optical calibration set (OCS) 24 for each measurement wavelength. That is, for each measurement wavelength, two optical calibration set (OCS) 24 are available, which - in the example of Figure 6 - are implemented by placing a respective auxiliary source 4 midway in between two primary detectors 3 (we note, however, that the optical calibration set (OCS) 24 could alternatively be implemented as was explained w.r.t. Figure 5, using an auxiliary detector 7 placed midway in between two primary sources 2). The two additional primary detectors 3 located at y-coordinates y₄ and y₆ thus enable two additional SDS (allowing thus different optical (mean) penetration depths into the tissue), which can be optically probed using primary radiation that is emitted from the respective primary source 2 located at y₇. As illustrated, a total of four different SDS (SDS1, SDS2, SDS3, SDS4) can thus be used for each of the N measurement wavelength (note that also only two primary sources 2 are shown, the concept can be easily expanded to more measurement wavelength by adding horizontal arrangement of sources 2, 4 and detectors 3, as has been illustrated in Figure 3). Due to the two OCS 24 positioned at y2 and y5, respectively, for each measurement wavelength and for each of the four SDS employed, a respective optical calibration measurement can be done using the secondary radiation emitted by the respective auxiliary source 4, as was explained above. Thus, based on secondary intensities Iₛᵢ(yᵢ) measured by the respective detector 3 at location yᵢ, it is possible to calculate the desired wavelength specific correction factors cᵢ(λₘᵢ, SDSⱼ)for each measurement wavelength and each SDS employed. Of course, the concept presented in Figure 6 can also be easily implemented by inversing sources 2,4 and detectors 3, as was explained w.r.t. Figure 5.

Figure 7 presents a more realistic (but still schematic) illustration of a possible implementation of an optical sensor 1 according to the invention, taking into consideration realistic sizes of photodiodes 14 employed as detectors 3 and LEDs 13 employed as light sources 2, 4. As indicated by the small circles, there are a total of eight separate LEDs used as primary light sources 2, each LED emitting one of the eight measurement wavelengths λₘ₁..λₘ₈. As shown, the location of each emission is spatially limited in the y-direction by a respective slit aperture. The dotted lines show the optical path of λₘ₈ for the two illustrated SDS3 and SDS4, as projected onto the xy-plane (note that there will be substantial penetration in the z-direction, however). The auxiliary light sources 4 shown on the left can be used to obtain correction factors for these two optical measurements with λₘ₈, using the (primary) detectors 3a and 3b, with which also primary intensities have been measured using λₘ₈. The dashed lines in Figure 7 illustrate the same for λₘ₂ and for the two illustrated SDS1 and SDS2. The auxiliary light sources 4 in the center can be used to calculate correction factors for these two optical measurements with λₘ₂, using the (primary) detectors 3c and 3d, with which also primary intensities have been measured using λₘ₂. As there are only four auxiliary light sources present at the left and in the center, respectively, there are only four different auxiliary wavelengths λₐ₁..λₐ₄ employed. However, using interpolation techniques, respective correction factors cᵢ(λₘᵢ) can be calculated for all eight measurement wavelengths λₘ₁..λₘ₈ and also for each SDS employed. We note that in this particular design, the respective overlap 21 of the respective mean optical paths 20 of measurement and auxiliary wavelengths are just below the respective detector (in the illustration 3a, 3b, 3c, 3d). Hence, if a tissue speckle is present below one of these detectors 3, its effect on the optical measurement can be successfully corrected.

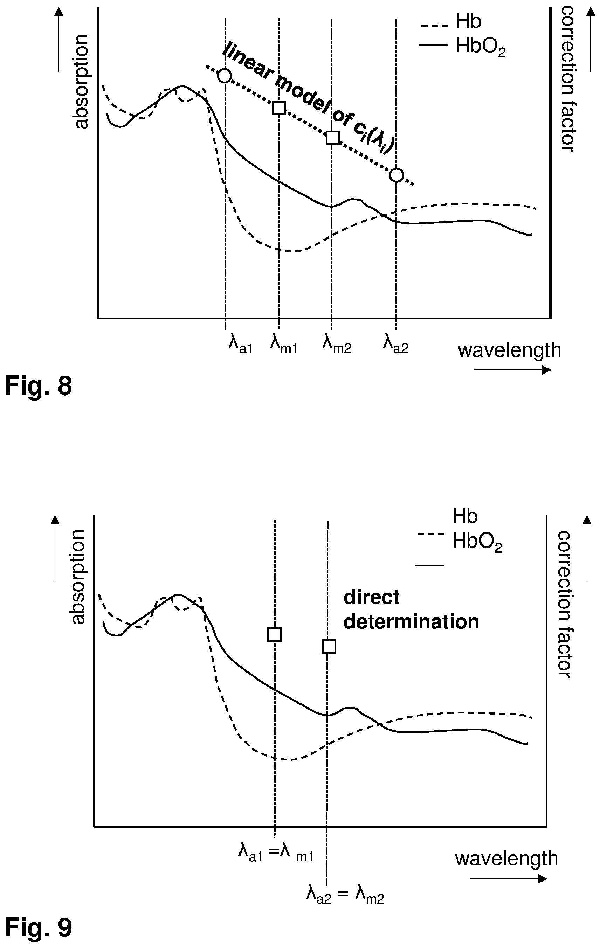

Figure 8 illustrates how wavelength specific correction factors cᵢ(λᵢ) can be derived by interpolation: the graph shows the absorption of two chromophores, namely Hb and HbO₂ as a function of wavelength. Two auxiliary wavelengths λₐ₁ and λₐ₂ are employed, which do not match the two measurement wavelengths λₘ₁ and λₘ₂ which are employed in a measurement of the concentration of Hb and HbO₂ in the tissue. However, based on secondary intensities measured for λₐ₁ and λₐ₂, it is possible to adapt a linear model (dotted line), i.e., choosing a best-fit linear curve, for a necessary correction factor cᵢ(λᵢ). Based on the adapted model, wavelength specific correction factors c₁(λₘ₁) and c₂(λₘ₂) can be calculated and applied for correcting primary intensities that have been measured using λₘ₁ and λₘ₂. As is visible from a comparison of the curves, the correction factors are thus determined in the spectral range relevant for the optical measurement.

Figure 9 presents a similar graph as Figure 8 but illustrates the case when a respective auxiliary wavelength λₐᵢ = λₘᵢ matches a corresponding measurement wavelength λₘᵢ. In this case, no extrapolation is necessary. Instead, the specific correction factors cᵢ(λᵢ), which is calculated from measured secondary intensities resulting from the respective auxiliary wavelength λₐᵢ, can be directly determined and used for correcting measured primary intensities, which result from an emission of the corresponding measurement wavelength λₘᵢ.

Finally, we note that (as in any optical system) the roles of sources and detectors may be interchanged, and the wavelengths for the sources and/or the characteristics of the optical filters employed may be adapted according to the application. Such adaptations or design transformations are obvious to a person skilled in the art and may thus be applied to the optical sensor as disclosed herein.

In summary, for improving the accuracy and reliability of an optical measurement performed with an optical sensor 1, which may be designed as an optical NIRS-sensor in particular, it is suggested to determine at least two different wavelength specific correction factors (c₁(λₘ₁), c₂(λₘ₂)). The correction factors are applied individually for correcting measurement results, in particular measured primary intensities, which have been obtained using a set of at least two distinct measurement wavelengths (λₘ₁ ≠ λₘ₂) forming a primary radiation that is propagating along respective primary optical paths 22. This concept is particularly useful for mitigating the detrimental effects of superficial absorbers on skin, which are illuminated by the primary radiation. The concept may be implemented by using separate auxiliary light sources 4 which emit an auxiliary light comprising at least two auxiliary wavelengths, which must not necessarily match the measurement wavelengths; or using at least one auxiliary detector 7 which also allows measurement of a secondary radiation that has propagated along a secondary optical path 23.

### List of reference numerals

- 1: sensor
- 2: primary light source
- 3: detector
- 4: auxiliary light source
- 5: medium (scatters light, e.g. human tissue, in particular nervous tissue)
- 6: primary detector
- 7: auxiliary detector
- 8: emission location
- 9: detection location
- 10: sensor head
- 11: flat cable
- 12: contact surface
- 13: LED
- 14: photodiode
- 15: flex-PCB
- 16: lower member
- 17: slit aperture
- 18: outer perimeter
- 19: electronic unit
- 20: mean optical path
- 21: overlap (of 20)
- 22: primary optical path
- 23: secondary optical path
- 24: optical calibration set (OCS)
- 25: stiff PCB

## Claims

1. **Method for quantitatively determining at least one optical or physiological parameter in a medium (5)** using an optical sensor (1), in particular according to claim 15, the method comprising the following steps:
- irradiating the medium (5) with a primary radiation comprising at least two distinct measurement wavelengths (λₘ₁ ≠ λₘ₂) which are emitted by at least one primary light source (2);
- measuring primary intensities (Iₘ₁, Iₘ₂) of the primary radiation for each of said at least two measurement wavelengths (λₘ₁, λₘ₂) after said primary radiation has propagated through said medium (5) along a respective primary optical path (22);
- determining for each of the at least two measurement wavelengths (λₘ₁, λₘ₂) a wavelength specific correction factor (c₁(λₘ₁), c₂(λₘ₂));
- calculating an estimate of the at least one optical or physiological parameter based on said measured primary intensities (Iₘ₁, Iₘ₂) and based on said at least two wavelength specific correction factors (c₁(λₘ₁), c₂(λₘ₂)).

2. Method according to claim 1, wherein the method further comprises the following steps:
- irradiating the medium (5) with a secondary radiation comprising at least two distinct auxiliary wavelengths (λₐ₁ ≠ λₐ₂) which are emitted by at least one auxiliary light source (4);
- measuring secondary intensities (Iₛ₁, Iₛ₂) of the secondary radiation for each of said at least two auxiliary wavelengths (λₐ₁, λₐ₂) after said secondary radiation has propagated through said medium (5) along a respective secondary optical path (23);
- determining the wavelength specific correction factors (c₁(λₘ₁), c₂(λₘ₂)) based on said secondary intensities (Iₛ₁, Iₛ₂), which result from the auxiliary wavelengths (λₐ₁ ≠ λₐ₂) emitted by the at least one auxiliary light source (4);

3. Method according to claim 1 or claim 2, wherein the method further comprises the following steps:
- detecting a secondary radiation comprising the at least two measurement wavelengths (λₘ₁, λₘ₂) with at least one auxiliary detector (7), wherein the secondary radiation has traveled along a secondary optical path (23) that is, at least partially, different from the primary optical path (22) along which said primary radiation has traveled,
- in particular wherein said secondary radiation has been emitted by a primary source (2);
- measuring secondary intensities (Iₛ₁, Iₛ₂) of the secondary radiation using the at least one auxiliary detector (7) for each of said at least two measurement wavelengths (λₘ₁, λₘ₂) after said secondary radiation has propagated through said medium (5) along the secondary optical path (23);
- determining the wavelength specific correction factors (c₁(λₘ₁), c₂(λₘ₂)) based on said secondary intensities (Iₛ₁, Iₛ₂) measured with the at least one auxiliary detector (7);

4. Method according to any of the preceding claims,
- wherein at least one, in particular both, of the at least two distinct auxiliary wavelengths (λₐ₁, λₐ₂) is
- identical to (λₐ₁ = λₘ₁ and/or λₐ₂ = λₘ₂) or
- distinct from (λₐ₁ ≠ λₘ₁ and/or λₐ₂ ≠ λₘ₂) a respective one of the at least two measurement wavelengths (λₘ₁, λₘ₂) ,
- preferably wherein each of said at least two auxiliary wavelengths (λₐ₁, λₐ₂) varies by less than 30%, preferably by less than 10%, most preferably by less than 3%, from a corresponding one of the at least two measurement wavelengths (λₘ₁, λₘ₂) .

5. Method according to any of the preceding claims,
- wherein at least one of the wavelength specific correction factors (c₁(λₘ₁), c₂(λₘ₂)) is interpolated mathematically, based on the measured secondary intensities (Iₛ₁, Iₛ₂),
- in particular wherein the secondary intensities (Iₛ₁, Iₛ₂) are measured without using the measurement wavelength (λₘ₁/ λₘ₂), for which the corresponding correction factor (c₁(λₘ₁) / c₂(λₘ₂)) is interpolated.

6. Method according to any of the preceding claims,
- wherein, based on the at least two different secondary intensities (Iₛ₁, Iₛ₂), each measured for one of said two distinct auxiliary wavelengths (λₐ₁, λₐ₂), respectively, a model c(λ) describing the wavelength dependence of at least one correction factor c(λ) which affects said measured primary intensities (Iₘ₁, Iₘ₂) is adapted and
- the adapted model c(λ) is used to determine said secondary estimate,
- preferably wherein said at least two wavelength specific correction factors (c₁(λₘ₁), c₂(λₘ₂)) are calculated from said adapted model c(λ) for each of said at least two measurement wavelengths (λₘ₁, Xₘ₂),
- in particular wherein at least one of said at least two auxiliary wavelengths (λₐ₁, λₐ₂) is distinct from and thus does not match any of said at least two measurement wavelengths (λₘ₁, λₘ₂) .

7. Method according to any of the preceding claims,
wherein the at least two wavelength specific correction factors (ci, c₂) define a respective wavelength specific correction c₁(λₘ₁), c₂(λₘ₂) that is to be applied to the primary intensities (Iₘ₁, Iₘ₂) measured for each of said at least two measurement wavelengths (λₘ₁, Xₘ₂),
- in particular wherein the respective correction factor is a ratio of two secondary intensities (I_{s1,SDS1}(λₐ₁) / I_{s1,SDS2}(λₐ₁)) which have been measured using the same auxiliary wavelength (λₐ₁),
- in particular and using the same detectors (3) or primary light sources (2), with which the primary intensities (Iₘ₁, Iₘ₂) have been measured.

8. Method according to any of the preceding claims, wherein the at least two wavelength specific correction factors (c₁(λₘ₁), c₂(λₘ₂)) correct the calculated estimate with respect to
- a wavelength dependent coupling factor k(λ),
- in particular wherein the coupling factor k(λ) defines a loss of light occurring at a specific wavelength at an interface of said medium (5)
and/or
- an absorption or scattering spectrum inside the medium (5) which is wavelength dependent, and/or
- an optical obstruction which shows a wavelength dependent transmission or scattering spectrum.

9. Method according to any of the preceding claims,
- wherein for each of the at least two measurement wavelengths (λₘ₁, Xₘ₂), at least two different primary intensities (Iₘ₁₁,Iₘ₁₂; Iₘ₂₁,Iₘ₂₂) are measured using two different source-detector-separations (SDS1ₘ₁, SDS2ₘ₁; SDS1ₘ₂, SDS2ₘ₂), respectively,
- preferably wherein said estimate is calculated from respective ratios of said at least two different primary intensities (Iₘ₁₁/Iₘ₁₂, Iₘ₂₁/Iₘ₂₂).

10. Method according to the preceding claim,
- wherein the same two different source-detector-separations (SDS1ₘ₁=SDS1ₘ₂; SDS2ₘ₁=SDS2ₘ₂) are used for measuring said at least two different primary intensities (Iₘ₁₁,Iₘ₁₂; Iₘ₂₁,Iₘ₂₂) for each of the at least two measurement wavelengths (λₘ₁, λₘ₂),
- in particular using at least two primary light sources (2) located at a common emission location (8) and two primary detectors (6) located at two distinct source-detector-separations (SDS1ₘ₁=SDS1ₘ₂ ≠ SDS2ₘ₁=SDS2ₘ₂) from said common emission location (8).

11. Method according to any of the preceding claims 2 to 10, wherein one of the at least one auxiliary light sources (4) is located
- equidistant from two primary detectors (6),
- in particular such that for an ideal homogenous medium (5), the determined at least two wavelength specific correction factors (c₁(λₘ₁), c₂(λₘ₂)) would be equal,
or
- non-equidistant from the two primary detectors (6a, 6b).

12. Method according to any one of the preceding claims,
- wherein at least one primary detector (6) is employed for measuring said primary intensities (Iₘ₁, Iₘ₂) and
- wherein said at least one auxiliary light source (4) is located closer to said at least one primary detector (6) than to said at least one primary light source (2),
- in particular such that a maximum source-detector-separation measurable with the auxiliary light source (4) is smaller than a maximum source-detector-separation measurable with said at least one primary light source (2) .

13. Method according to any one of the preceding claims,
- wherein each time an updated value is determined for said estimate, a calibration measurement is performed beforehand using the secondary radiation to determine updated values of the at least two correction factors (c₁(λₘ₁), C₂(λₘ₂)),
- preferably wherein a multitude of such calibration measurements are performed to obtain average values for said at least two correction factors (c₁(λₘ₁), c₂(λₘ₂)) prior to determining an updated value of the estimate.

14. Method according to any one of the preceding claim,
- wherein a calibration measurement is performed using the secondary radiation to calculate updated values of the at least two correction factors (ci, c₂) as soon as a movement of an optical sensor (1), which is used for determining said estimate, is detected,
- in particular wherein the movement is detected by the optical sensor (1) itself, for example based on the read-out of an accelerometer or of an optical detector.

15. **Optical sensor (1),** in particular configured as a NIRS-sensor or as an oximeter, for measuring an optical or physiological parameter in a medium (5) such as tissue, the sensor (1) comprising,
- at least one primary light source (2) for emitting a primary radiation comprising at least two distinct measurement wavelengths (λₘ₁, Xₘ₂),
- at least one primary detector (3) for detecting primary intensities (Iₘ₁, Iₘ₂) of the primary radiation after said primary radiation has propagated through said medium (5) along a respective primary optical path (22), and
- an electronic unit (19) configured for computing an estimate of the at least one optical or physiological parameter based on said measured primary intensities, **characterized in that** the sensor (1) features
- at least one auxiliary light source (4) for emitting a secondary radiation comprising at least two distinct auxiliary wavelengths (λₐ₁ ≠ λₐ₂) and/or
- at least one auxiliary detector (7) capable of measuring secondary intensities (Iₛ₁, Iₛ₂) of a secondary radiation comprising the at least two distinct measurement wavelengths (λₘ₁, λₘ₂) after said secondary radiation has propagated through said medium (5) along a respective secondary optical path (23) which is different from said primary optical path (22),
- and the electronic unit (19) is configured to implement a method according to one of the preceding claims.
